# EUROPÄISCHE PATENTSCHRIFT

(11) **EP 0 820 435 B1**
(45) Veröffentlichungstag und Bekanntmachung des Hinweises auf die Patenterteilung: **16.05.2001**
(21) Anmeldenummer: 96910014.8
(22) Anmeldetag: 25.03.1996
(51) Int. Cl.: C07C 249/04, C07C 249/14

(54) **VERFAHREN ZUR HERSTELLUNG VON WEITGEHEND ISOMERENREINEN ALPHA-BISOXIMEN**
METHOD OF PREPARING ESSENTIALLY PURE ISOMERS OF ALPHA-BIS-OXIMES
PROCEDE DE PREPARATION D'ISOMERES SENSIBLEMENT PURS D'ALPHA-BIS-OXIMES

(30) Priorität: 08.04.1995 DE 19513388
(43) Veröffentlichungstag der Anmeldung: 28.01.1998
(73) Patentinhaber: BASF AKTIENGESELLSCHAFT, 67056 Ludwigshafen (DE)
(72) Erfinder: MÜLLER, Ruth, D-67159 Friedelsheim (DE); REMY, Benoit, D-67435 Neustadt (DE); BAYER, Herbert, D-68159 Mannheim (DE); GÖTZ, Norbert, D-67547 Worms (DE); SAUTER, Hubert, D-68167 Mannheim (DE)
(86) Internationale Anmeldenummer: EP9601306
(87) Internationale Veröffentlichungsnummer: WO9632373

(56) Entgegenhaltungen:
- EP-A- 0 435 687
- US-A- 4 158 015
- JOURNAL OF ORGANIC CHEMISTRY, Bd. 20, 1955, EASTON US, Seiten 1491-1495, XP002009319 C. R. HAUSER ET AL.: "Syn-anti isomerism of p-chlorobenzaldoxime with boron fluoride"
- "Houben-Weyl Methoden der Organischen Chemie vol X/4" 1968 , E. MÜLLER , STUTTGART (DE) XP002009320 siehe Seite 292, Zeile 6 - Seite 294, Zeile 4 und Seite 293, Referenz 13 & BERICHTE DER DEUTSCHEN CHEMISCHEN GESELLSCHAFT, Bd. 62, 1929, WEINHEIM DE, Seiten 1839-1846, W. HIEBER ET AL.:

## Beschreibung

Die vorliegende Erfindung betrifft ein Verfahren zur Herstellung von weitgehend isomerenreinen α-Bisoximen der Formel Ia

R¹O-N=CR²-CR³=N-OR⁴ Ia

in denen die Gruppen R¹O- und R² an der N=C Bindung *cis* zueinander stehen und in denen die Reste die folgende Bedeutung haben:
- R¹: ein C-organischer Rest;
- R²: Wasserstoff, Cyano, Nitro, Hydroxy, Amino, Halogen oder ein organischer Rest, welcher direkt oder über ein Sauerstoff-, Schwefel- oder ein Stickstoffatom an das Gerüst gebunden sein kann;
- R³: Wasserstoff, Cyano, Nitro, Hydroxy, Amino, Halogen, Alkyl, Halogenalkyl, Alkoxy, Halogenalkoxy, Alkylthio, Alkylamino, Dialkylamino oder Cycloalkyl;
- R⁴: Wasserstoff.

α-Bisoxime sind aus der Literatur als Zwischenprodukte und als Wirkstoffe zur Bekämpfung von tierischen Schädlingen oder Schadpilzen bekannt (WO-A 95/18,789; WO-A 95/21,153; WO-A 95/21,154; WO-A 95/21,156; WO-A 96/07,633; WO-A 96/16,030.

Die beschriebenen Wirkstoffe entfalten, je nach der Konfiguration der Doppelbindung der Gruppe R¹O-N=CR²- im allgemeinen unterschiedliche Wirkung, wobei die Wirkung derjenigen Verbindungen üblicherweise höher ist, in denen die Gruppen R¹O- und R² an der N=C Bindung *cis* zueinander stehen. Dem Rechnung tragend wird in der genannten Literatur vorgeschlagen, die Isomeren auf herkömmlichen Wegen (z.B. durch Chromatographie) voneinander zu trennen. Die Abtrennung des gewünschten Isomers aus einer Mischung von Isomeren hat zum Nachteil, daß ein nicht unbeträchtlicher Teil teurer Zwischen- oder Endprodukte ungenutzt bleiben, weil sie in der "falschen" Konfiguration vorliegen.

Die Isomerisierung von Monooximen wird in J. Org. Chem. 20 1491 (1955) und US-A 4,158,015 beschrieben. Des weiteren ist aus EP-A 435 687 ein Verfahren zur Isomerisierung von Monooximethern in Gegenwart von Lewis Säuren beschrieben, wobei der sterisch anspruchsvollere (Phenyl)rest nach der Isomerisierung transständig zur Alkoxygruppe im Oximteil sitzt.

Aus der Literatur sind Verfahren zur Isomerisierung von α-Bisoximen bekannt, deren Nachteil darauf beruht, daß sie entweder auf Oxime mit bestimmten (stabilen) Resten beschränkt sind und/oder unselektiv und/oder nur mit schlechten Ausbeuten verlaufen [DE-A 29 08 688; J. General Chem. USSR 58/1, 181 (1988); Tetrahedron 41/22, 5181 (1985); J. Org. Chem. USSR 20, 135 (1984); J. Org. Chem. USSR 27/1, 97 (1991); J. Phys. Chem. 91/26, 6490 (1987); Recl. Trav. Chim. Pays-Bas 111/2, 79 (1992)].

Außerdem wird in der Literatur die Überführung von Bis-Oximen in ein bestimmtes Isomer unter Säurekatalyse beschrieben [Synth. Reakt. Inorg. Met.-Org. Chem. 18(10), 975 (1988); Synth. Reakt. Inorg. Met.-Org. Chem. 11(7), 621 (1981); Spectrosc. Lett. 23 (6), 713 (1990); Z. Anorg. Allg. Chem. 496, 197 (1983)]. Diese Verfahren haben jedoch den Nachteil, daß sie lediglich bei solchen Verbindungen angewendet werden können, deren Substituenten gegen Säuren stabil sind.

Desweiteren wird in der älteren Anmeldung WO-A 95/21,153 die Isomerisierung der Verbindung **A** zu **A'** in Ether mit HC1 mit 75 %-iger Ausbeute beschrieben.

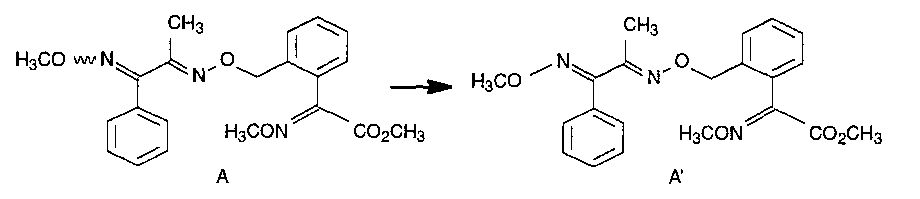

Der vorliegenden Erfindung lag ein generell anwendbares Verfahren zur Herstellung von weitgehend isomerenreinen α-Bisoximen der Formel Ia

R¹O-N=CR²-CR³=N-OR⁴ Ia

in denen die Gruppen R¹O- und R² an der N=C Bindung *cis* zueinander stehen, als Aufgabe zugrunde, welches insbesondere auch auf solche Verbindungen anwendbar ist, in denen die Substituenten R¹, R² und R³ nicht gegen Säuren stabil sind oder nicht gegen Säuren stabile Gruppen enthalten.

Demgemäß wurde ein Verfahren zur Herstellung von weitgehend isomerenreinen α-Bisoximen der Formel Ia

R¹O-N=CR²-CR³=N-OR⁴ Ia

in denen die Gruppen R¹O- und R² an der N=C Bindung *cis* zueinander stehen, gefunden, welches dadurch gekennzeichnet ist, daß man eine Mischung der Isomere der α-Bisoxime Ia und Ib 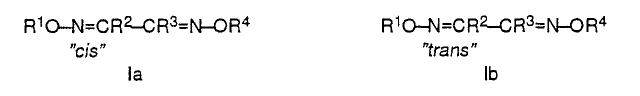 in einem aliphatischen oder aromatischen Kohlenwasserstoff mit einer Lewis Säure behandelt.

Nach dem erfindungsgemäßen Verfahren können die weitgehend isomerenreinen α-Bisoxime der Formel Ia auch dann erhalten werden, wenn man ein α-Bisoxim Ib, in dem die Gruppen R¹O- und R² an der N=C Bindung trans zueinander stehen, in einem aliphatischen oder aromatischen Kohlenwasserstoff mit einer Lewis Säure behandelt.

Geeignet sind beispielsweise aliphatische Kohlenwasserstoffe wie Pentan, Hexan, Heptan, Cyclohexan und Petrolether und aromatische Kohlenwasserstoffe wie Benzol, Toluol, o-, m- und p-Xylol. Besonders bevorzugt sind die aliphatischen Kohlenwasserstoffe: Pentan, Hexan, Heptan, Cyclohexan und Petrolether und die aromatischen Kohlenwasserstoffe: Toluol, o-, m- und p-Xylol. Es können auch Gemische der genannten Lösungsmittel verwendet werden.

Die Umsetzungstemperatur ist von der Art der Substituenten der α-Bisoxime abhängig. Im allgemeinen wird die Isomerisierung bei Temperaturen von mindestens -40°C durchgeführt. Bei Temperaturen von über 150°C kann bereits eine temperaturbedingte Zersetzung der Bisoxime eintreten. Diese temperaturbedingte Zersetzung hängt jedoch im wesentlichen von der Art und Stabilität der Substituenten ab, so daß in Fällen von stabilen Bisoximen die Reaktion auch bei höheren Temperaturen durchgeführt werden kann.

Diese Umsetzung erfolgt daher üblicherweise bei Temperaturen von - 30°C bis 140°C, vorzugsweise -10°C bis 120°C.

Grundsätzlich können bei der erfindungsgemäßen Umsetzung alle Lewis Säuren verwendet werden, die im gewählten Lösungsmittel so stabil sind, daß keine Protonensäuren freigesetzt werden.

Als Lewis Säuren können daher im allgemeinen die Halogenide eines Halbmetalls oder Metalls der 3. oder 4. Hauptgruppe oder eines Übergangsmetalls verwendet werden. Bevorzugt werden Halogenide des Bors, Aluminiums, Zinns, Zinks, Eisens oder Titans verwendet. Als Halogenide eignen sich dabei besonders die Fluoride, Chloride oder Bromide. Beispiele für übliche Lewis Säuren, die nach dem erfindungsgemäßen Verfahren Verwendung finden können, sind AlCl₃, AlBr₃, FeCl₃, BBr₃, BCl₃, BF₃, SnCl₄, ZnCl₂, ZnBr₂ oder TiCl₄.

Im allgemeinen ist es ausreichend, wenn die Lewis Säuren in katalytischen Mengen verwendet werden, wobei mit steigender Menge an Lewis Säure die Umsetzungsgeschwindigkeit steigen kann. Demgemäß sind üblicherweise Mengen von 0,1 bis 500 Mol-% der Lewis Säure (bezogen auf die Menge an Bisoxim Ib oder der Mischung der Bisoxime Ia und Ib) bereits ausreichend. Größere Mengen stören die Umsetzung nicht, sind aus wirtschaftlicher Sicht im allgemeinen jedoch nicht erforderlich und im Hinblick auf eine großtechnische Anwendung des Verfahrens (sicherheitstechnische Aspekte und Fragen der Umweltbelastung) nicht wünschenswert. Demgemäß empfiehlt es sich, die Lewis Säuren üblicherweise in Mengen von 0,5 Mol-% bis 300 Mol-%, vorzugsweise 1 Mol-% bis 150 Mol-%, insbesondere 10 Mol-% bis 80 Mol-%, zu verwenden.

Entsprechend den vorstehend beschriebenen Verfahrensmaßnahmen ist es insbesondere möglich weitgehend isomerenreine α-Bisoxime der Formel Ia' herzustellen,

R¹O-N=CR²-CR³=N-OR⁴ Ia'

in denen die Gruppen R¹O- und R² und die Gruppen R³ und -OR⁴ an den N=C Bindungen jeweils *cis* zueinander stehen, indem man eine Mischung der Isomere der α-Bisoxime Ia' und Ib' 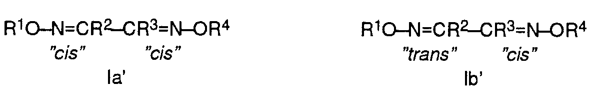 in einem aliphatischen oder aromatischen Kohlenwaserstoff mit einer Lewis Säure behandelt.

Nach dem erfindungsgemäßen Verfahren können die weitgehend isomerenreinen α-Bisoxime Ia' auch dann erhalten werden, wenn man ein α-Bisoxim Ib' in einem aliphatischen oder aromatischen Kohlenwasserstoff mit einer Lewis-Säure behandelt.

Die Reaktionsgemische werden in üblicher Weise aufgearbeitet, z.B. durch Mischen mit Wasser, Trennung der Phasen und gegebenenfalls chromatographische Reinigung der Rohprodukte. Die Endprodukte fallen z.T. in Form zäher Öle an, die unter vermindertem Druck und bei mäßig erhöhter Temperatur von flüchtigen Anteilen befreit oder gereinigt werden. Sofern die Endprodukte als Feststoffe erhalten werden, kann die Reinigung auch durch Umkristallisieren oder Digerieren erfolgen.

Das erfindungsgemäße Verfahren ist breit anwendbar. Es ist nach diesem Verfahren insbesondere möglich, weitgehend isomerenreine α-Bisoxime Ia bzw. Ia' bereitzustellen, die Gruppen tragen, welche unter sauren Reaktionsbedingungen instabil sind oder welche Komplexe mit Lewis Säuren bilden können, beispielsweise durch Halogenatome oder Ether- oder Estergruppen substituierte Verbindungen (vgl. Tabelle 1).

Unerwarteterweise erhält man nach dem vorstehend beschriebenen Verfahren von vier möglichen Isomeren der α-Bisoxime überwiegend das Isomer Ia bzw. Ia'. Das erfindungsgemäße Verfahren bietet Reaktionsbedingungen, unter denen im allgemeinen bevorzugt eine Isomerisierung der R¹O- substituierten N=C-Bindung erfolgt, während eine Isomerisierung der R⁴O- substituierten N=C-Doppelbindung weitgehend vermieden werden kann {vgl. Tabelle 2 und Tabelle 3 ([i^{e}] : I^{x})}.

Das erfindungsgemäße Verfahren eignet sich insbesondere zur Herstellung der weitgehend isomerenreinen α-Bisoxime, die in der eingangs zitierten Literatur als Zwischenprodukte und als Wirkstoffe zur Bekämpfung von tierischen Schädlingen oder Schadpilzen beschrieben sind. Demgemäß umfaßt der Begriff "C-organischer Rest" und der Begriff "organischer Rest" insbesondere die in dieser Literatur im allgemeinen und im besonderen angegebenen Bedeutungen.

Beispiele:
Allgemeine Vorgehensweise bei der Umsetzung von Isomerengemischen der α-Bisoxime
   a) **X** mol des Isomerengemischs der α-Bisoxime [**I'**; Isomerenverhältnis (Ia':Ib'): **i°**] in **Y** ml des organischen Lösungsmittels [**LSGM**] wurden mit **Z** mol-% der Lewis Säure [**LS**] versetzt und für **t** Stunden bei einer Temperatur [**T**°C] gerührt. Nach der Aufarbeitung wurde mittels Gaschromatographie (**GC**), **HPLC** oder ¹H-NMR-Spektroskopie (**NMR**) das unter diesen Bedingungen erhaltene Isomerenverhältnis [**i**^{**e**}] (≡ Ia':Ib') untersucht. Die Ergebnisse dieser Untersuchungen sind in der Tabelle 1 zusammengestellt.
   b) In weiteren Versuchen wurden **X** mol des Isomerengemischs der α-Bisoxime I' [R¹ = CH₃, R² = C₆H₅, R³ = CH₃, R⁴ = H; Isomerenverhältnis (Ia':Ib'): **i°**] in Y ml des organischen Lösungsmittels [**LSGM**] mit **Z** mol-% der Lewis Säure [**LS**] versetzt und für t Stunden bei einer Temperatur [**T**°C] gerührt. Nach der Aufarbeitung wurde mittels Gaschromatographie (**GC**), **HPLC** oder ¹H-NMR-Spektroskopie (**NMR**) das unter diesen Bedingungen erhaltene Isomerenverhältnis [**i**^{**e**}]:I^{x} {[**i**^{**e**}] ≡ Ia':Ib'} untersucht (I^{x}: Menge eines weiteren Isomers noch nicht bekannter Stereochemie). Die Ergebnisse dieser Untersuchungen sind in der Tabelle 2 zusammengestellt.
   c) In weiteren Versuchen gemäß Z. Anorg. Chem. 496, 197 (1983) wurden **X** mol des Isomerengemischs der α-Bisoxime I' (R¹ = CH₃, R² = C₆H₅, R³ = CH₃, R⁴ = H; Isomerenverhältnis (Ia':Ib'): **i**°] in **Y** ml des organischen Lösungsmittels [**LSGM**] mit HCl-Gas gesättigt und für **t** Stunden bei einer Temperatur [**T**°C] gerührt. Nach der Aufarbeitung wurde mittels Gaschromatographie (**GC**), **HPLC** oder ¹H-NMR-Spektroskopie (**NMR**) das unter diesen Bedingungen erhaltene Isomerenverhältnis [**i**^{**e**}]:I^{x} untersucht (I^{x}: Menge eines weiteren Isomers noch nicht bekannter Stereochemie; [**i**^{**e**}] **≡** Ia':Ib'). Die Ergebnisse dieser vergleichenden Untersuchungen sind in der Tabelle 3 zusammengestellt.

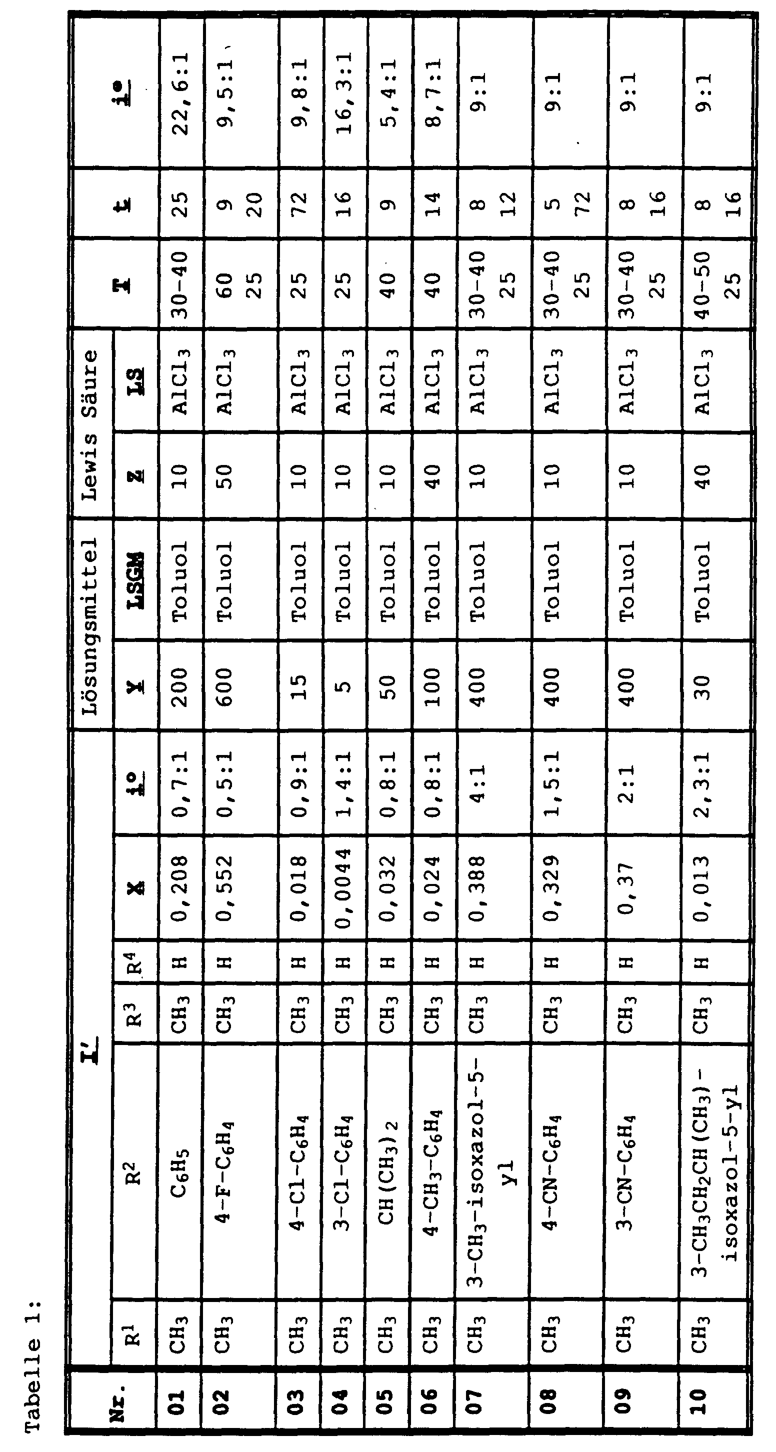

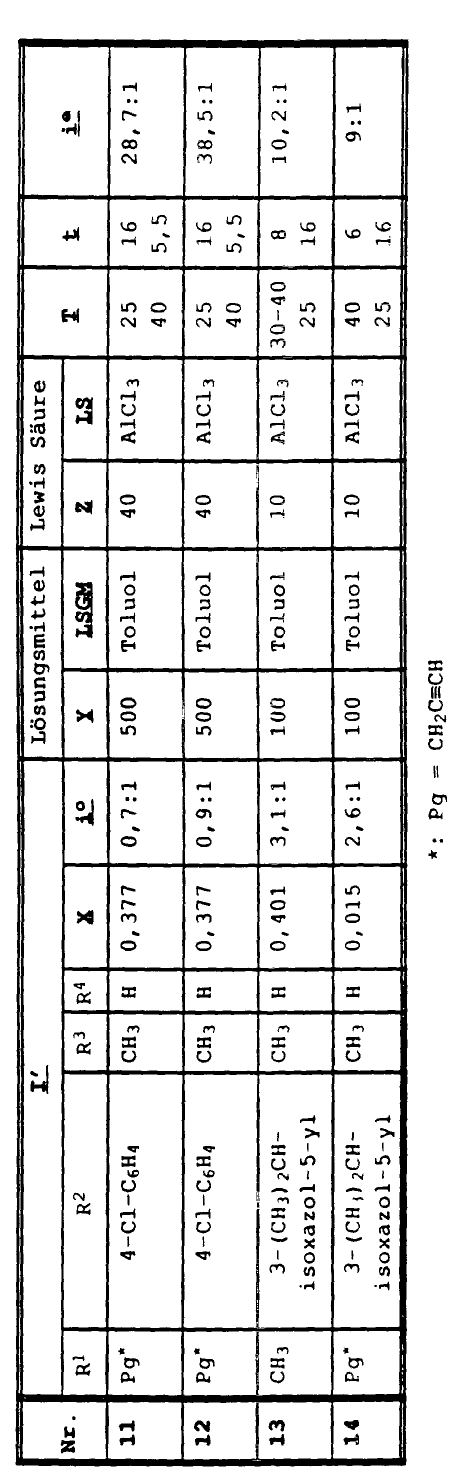

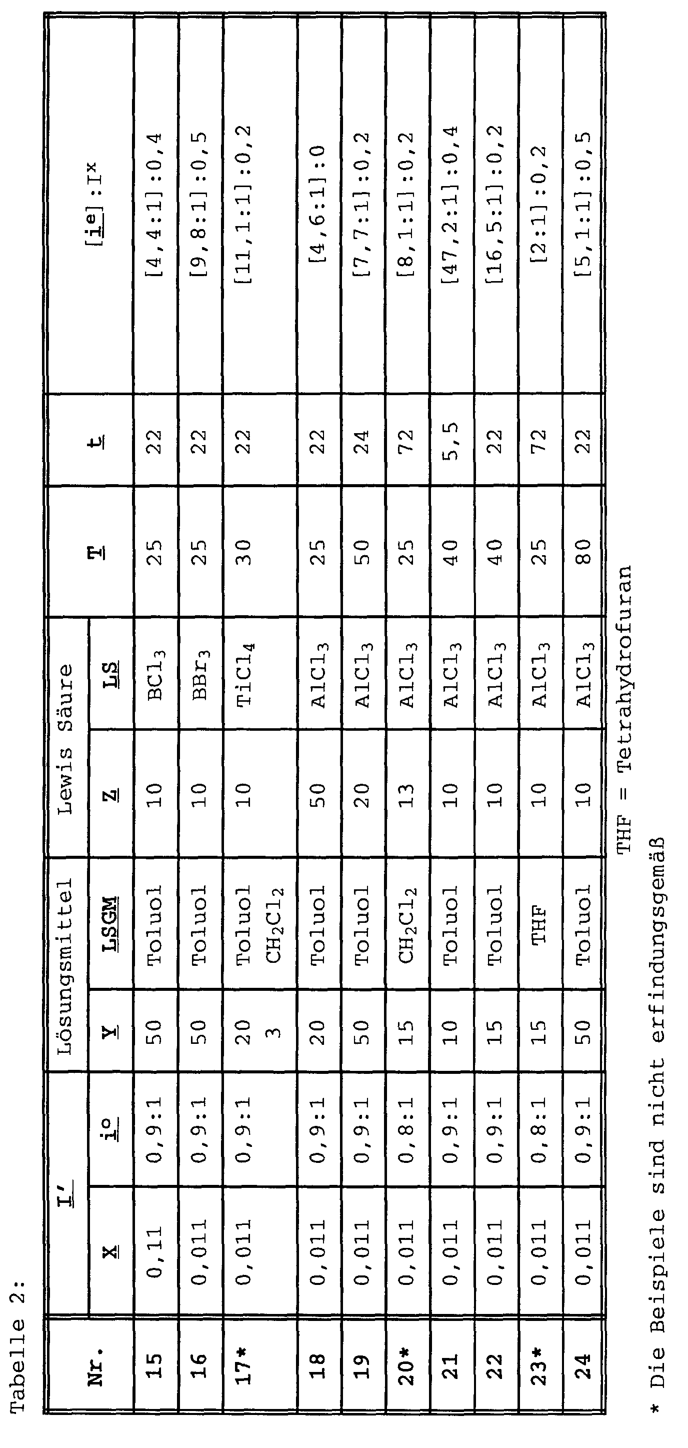

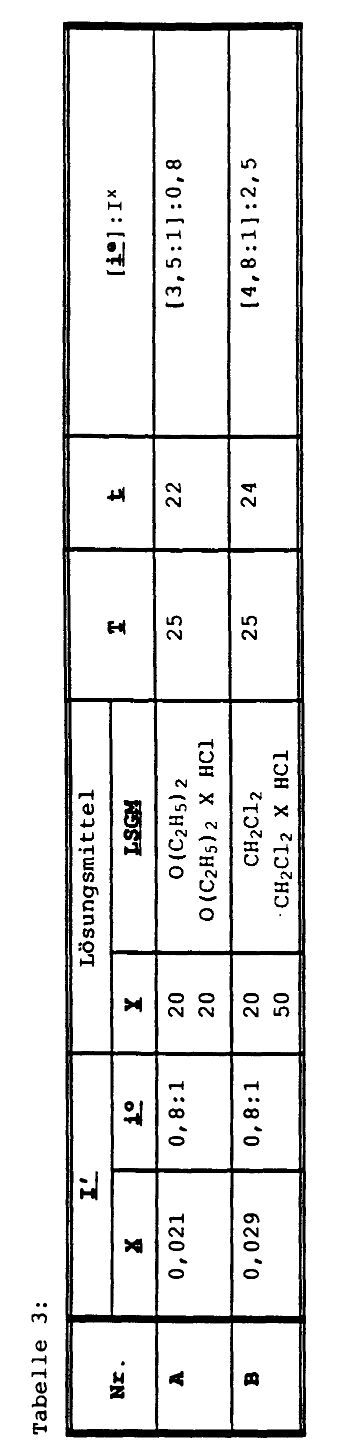

### Beispiel 01 (Tabelle 1): Herstellung von (E,E)-1-Phenyl-1-methoxyiminopropan-2-on-2-oxim

40 g (0,208 mol) (E,E/Z,E)-1-Phenyl-1-methoxyiminopropan-2-on-2-oxim (Isomerenverhältnis E,E : Z,E = 1 : 1,4) in 200 ml Toluol wurden mit 2,77 g AlCl₃ versetzt. Nach 25 h bei 30-40°C wurde das Reaktionsgemisch mit Essigsäure-ethyl-ester versetzt. Die Mischung wurde mit 2N Salzsäure gewaschen und getrocknet. Anschließend wurde das Lösungsmittel bei vermindertem Druck abdestilliert. Nach Kristallisation in n-Pentan erhielt man 32,5 g (81% d.Th.) der Titelverbindung als farblose Kristalle (Fp.: 160-162°C).
1H-NMR [CDCl₃/TMS; δ (ppm)]: 2,10 (s, 3H); 3,91 (s, 3H); 7,17 (m, 2H); 7,40 (m, 3H); 8,66 (s breit, OH)

### Beispiel 02 (Tabelle 1): Herstellung von (E,E)-1-(4-Fluorphenyl)-1-methoxyiminopropan-2-on-2-oxim

115,9 g (0,552 mol) (E,E/Z,E)-1-(4-Fluorphenyl)-1-methoxyiminopropan-2-on-2-oxim (Isomerenverhältnis E,E : Z,E = 1 : 1,9) in 600 ml Toluol wurden mit 36,7 g AlCl₃ versetzt. Nach 9 h bei 60°C und 20 h bei Raumtemperatur (ca. 25°C) wurde das Reaktionsgemisch in eine Mischung von Essigsäureethylester und Eiswasser gegeben. Die Mischung wurde mit 10%-iger Salzsäure versetzt und mit Essigsäureethylester extrahiert. Die organische Phase wurde gewaschen und getrocknet. Anschließend wurde das Lösungsmittel bei vermindertem Druck abdestilliert. Nach Kristallisation in n-Pentan erhielt man 86,4 g (75% d.Th.) der Titelverbindung als farblose Kristalle (Fp.: 156-157°C).
1H-NMR [CDCl₃/TMS; δ (ppm)]: 2,10 (s, 3H) ; 3,91 (s, 3H); 7,03-7,25 (m, 4H); 8,67 (s, OH)

### Beispiel 03 (Tabelle 1) : Herstellung von (E,E)-1-(4-Chlorphenyl)-1-methoxyiminopropan-2-on-2-oxim

4 g (0,018 mol) (E,E/Z,E)-1-(4-Chlorphenyl)-1-methoxyiminopropan-2-on-2-oxim (Isomerenverhältnis E,E : Z,E = 1 : 1,1) in 15 ml Toluol wurden mit 0,2 g AlCl₃ versetzt. Nach 72 h bei Raumtemperatur (ca. 25°C) wurde das Reaktionsgemisch mit Eiswasser versetzt. Die Mischung wurde mit tert.-Butyl-methylether extrahiert. Die organische Phase wurde mit 10%-iger Salzsäure und Wasser gewaschen und getrocknet. Anschließend wurde das Lösungsmittel bei vermindertem Druck abdestilliert. Nach Kristallisation in n-Pentan erhielt man 3,4 g (85% d.Th.) der Titelverbindung als farblose Kristalle (Fp.: 174-176°C).
1H-NMR [CDCl₃/TMS; δ (ppm)] : 2,13 (s, 3H); 3,92 (s, 3H); 7,12 (d, 2H); 7,36 (d, 2H); 8,42 (s breit, OH)

### Beispiel 07 (Tabelle 1): Herstellung von (Z,E)-1-(3-Methylisoxazol-5-yl)-1-methoxyiminopropan-2-on-2-oxim

76,4 g (0,388 mol) (E,E/Z,E)-1-(3-Methylisoxazol-5-yl)-1-methoxyiminopropan-2-on-2-oxim (Isomerenverhältnis Z,E : E,E = 4 : 1) in 400 ml Toluol wurden mit 5,2 g AlCl₃ versetzt. Nach 8 h bei 30-40°C und weiteren 12 h bei Raumtemperatur (ca. 25°C) wurde das Reaktionsgemisch zu einer Mischung aus 100 ml Essigsäureethylester und 200 ml 2N Salzsäure gegeben. Die Mischung wurde mit Essigsäureethylester extrahiert. Die organische Phase wurde gewaschen und getrocknet. Anschließend wurde das Lösungsmittel bei vermindertem Druck abdestilliert. Nach Kristallisation in n-Pentan erhielt man 65 g (85% d.Th.) der Titelverbindung als gelbliche Kristalle in einer Reinheit von 90%.
1H-NMR [CDCl₃/TMS; δ (ppm)] : 2,18 (s, 3H); 2,35 (s, 3H); 4,07 (s, 3H) ; 6,57 (s, 1H) ; 9,67 (s breit, OH)

### Beispiel 08 (Tabelle 1) : Herstellung von (E,E)-1-(4-Cyanophenyl)-1-methoxyiminopropan-2-on-2-oxim

71,4 g (0,329 mol) (E,E/Z,E)-1-(4-Cyanophenyl)-1-methoxyiminopropan-2-on-2-oxim (Isomerenverhältnis E,E : Z,E = 1,5 : 1) in 400 ml Toluol wurden mit 4,4 g AlCl₃ versetzt. Nach 5 h bei 35-40°C und weiteren 72 h bei Raumtemperatur (ca. 25°C) wurde das Reaktionsgemisch zu einer Mischung aus Essigsäureethylester und 2N Salzsäure gegeben. Nach Extraktion mit Essigsäureethylester wurde die organische Phase gewaschen und getrocknet. Anschließend wurde das Lösungsmittel bei vermindertem Druck abdestilliert. Nach Kristallisation in n-Pentan/Methanol erhielt man 60,6 g (85% d.Th.) der Titelverbindung als gelbliche Kristalle (Fp.: 165-170°C).
1H-NMR [CDCl₃/TMS; δ (ppm)]: 2,13 (s, 3H); 3,92 (s, 3H); 7,27 (d, 2H); 7,66 (d, 2H); 8,75 (s breit, OH)

### Beispiel 09 (Tabelle 1): Herstellung von (E,E)-1-(3-Cyanophenyl)-1-methoxyiminopropan-2-on-2-oxim

80,2 g (0,37 mol) (E,E/Z,E)-1-(3-Cyanophenyl)-1-methoxyiminopropan-2-on-2-oxim (Isomerenverhältnis E,E : Z,E = 2 : 1) in 400 ml Toluol wurden mit 4,9 g AlCl₃ versetzt. Nach 8 h bei 30-40°C und weiteren 16 h bei Raumtemperatur (ca. 25°C) wurde das Reaktionsgemisch zu einer Mischung aus Essigsäureethylester und 2N Salzsäure gegeben. Nach Extraktion mit Essigsäureethylester wurde die organische Phase gewaschen und getrocknet. Anschließend wurde das Lösungsmittel bei vermindertem Druck abdestilliert. Nach Kristallisation in n-Pentan/Methanol erhielt man 67,1 g (84% d.Th.) der Titelverbindung als gelbliche Kristalle (Fp.: 163-166°C).
1H-NMR [CDCl₃/TMS; δ (ppm)] : 2,13 (s, 3H) ; 3,93 (s, 3H); 7,40-7,66 (m, 4H); 8,54 (s breit, OH)

### Beispiel 25: Herstellung von (E,E/Z,E)-1-Phenyl-1-methoxyiminopropan-2-on-2-oxim (Ausgangsprodukt)

100 g (0,614 mol) 1-Phenyl-1,2-propandion-2-E-oxim in 200 ml Methanol und 144 g Pyridin wurden mit einer Lösung aus 77 g (0,922 mol) O-Methylhydroxylamin-Hydrochlorid und 200 ml Methanol versetzt. Nach 24 h bei Raumtemperatur (ca. 25°C) wurde das Lösungsmittel bei vermindertem Druck entfernt. Der so erhaltene Rückstand wurde in tert.-Butyl-methylether aufgenommen und mit 2N Salzsäure versetzt. Die wäßrige Phase wurde erneut mit tert.-Butyl-methylether extrahiert. Aus den vereinigten organischen Phasen erhielt man nach Waschen, Trocknen und Entfernen des Lösungsmittels das Gemisch der Isomere E,E:Z,E in einem Verhältnis von 1:1,4 (GC-Bestimmung) in einer Reinheit von 90-95%.
1H-NMR [CDCl₃/TMS; δ (ppm)] : 2,05/2,10 (2s, 1H,1H^{*}); 3,91/3,97 (2s, 1H,1H^{*}); 7,18/7,38/7,61 (3m, 5H,5H^{*}); 9,14 (s breit, OH,OH^{*})

In entsprechender Weise konnten beispielsweise die in Tabelle 4 aufgeführten Verbindungen Ia' erhalten werden:

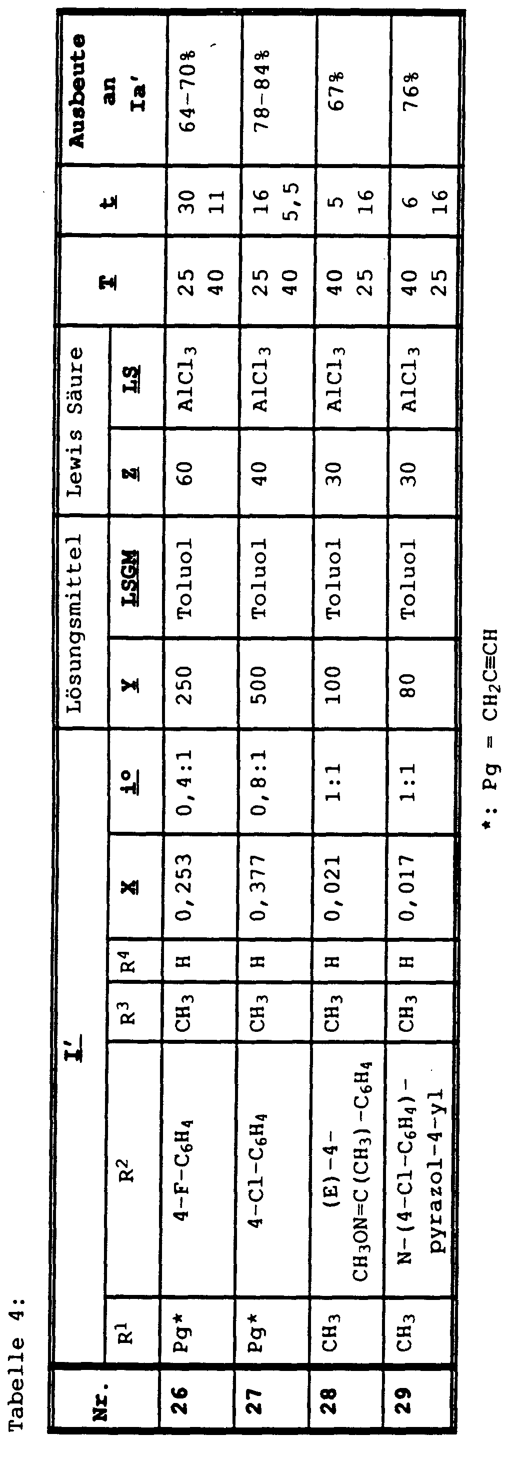

## Patentansprüche

1. Verfahren zur Herstellung von weitgehend isomerenreinen α-Bisoximen der Formel Ia
R¹O-N=CR²-CR³=N-OR⁴ Ia
in denen die Gruppen R¹O- und R² an der N=C Bindung *cis* zueinander stehen und in denen die Reste die folgende Bedeutung haben:
R¹ ein C-organischer Rest;
R² Wasserstoff, Cyano, Nitro, Hydroxy, Amino, Halogen oder ein organischer Rest, welcher direkt oder über ein Sauerstoff-, Schwefel- oder ein Stickstoffatom an das Gerüst gebunden sein kann;
R³ Wasserstoff, Cyano, Nitro, Hydroxy, Amino, Halogen, Alkyl, Halogenalkyl, Alkoxy, Halogenalkoxy, Alkylthio, Alkylamino, Dialkylamino oder Cycloalkyl;
R⁴ Wasserstoff,
dadurch gekennzeichnet, daß man eine Mischung der Isomere der α-Bisoxime Ia und Ib 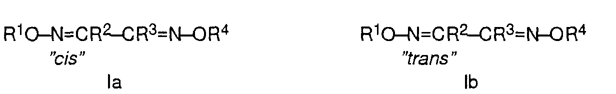 in einem aliphatischen oder aromatischen Kohlenwasserstoff mit einer Lewis Säure behandelt.

2. Verfahren gemäß Anspruch 1, dadurch gekennzeichnet, daß man ein α-Bisoxim Ib gemäß Anspruch 1, in dem die Gruppen R¹O- und R² an der N=C Bindung *trans* zueinander stehen, behandelt.

3. Verfahren zur Herstellung von weitgehend isomerenreinen α-Bisoximen der Formel Ia'
R¹O-N=CR²-CR³=N-OR⁴ Ia'
in denen die Gruppen R¹O- und R² und die Gruppen R³ und -OR⁴ an den N=C Bindungen jeweils *cis* zueinander stehen nach Anspruch 1, dadurch gekennzeichnet, daß man eine Mischung der Isomere der α-Bisoxime Ia' und Ib' 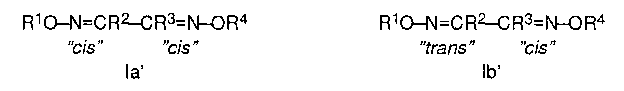 in einem aliphatischen oder aromatischen Kohlenwasserstoff mit einer Lewis Säure behandelt.

4. Verfahren gemäß Anspruch 3, dadurch gekennzeichnet, daß man ein α-Bisoxim Ib' gemäß Anspruch 3, in dem die Gruppen R¹O- und R² an der N=C Bindung trans und die Gruppen R³ und -OR⁴ an der C=N Bindung *cis* zueinander stehen, behandelt.

5. Verfahren nach Anspruch 1, 2, 3 oder 4, dadurch gekennzeichnet, daß man die Umsetzung bei Temperaturen von -40°C bis 150°C durchführt.

6. Verfahren nach Anspruch 1, 2, 3 oder 4, dadurch gekennzeichnet, daß man als Lewis Säure ein Halogenid eines Halbmetalls oder Metalls der 3. oder 4. Hauptgruppe oder eines Übergangsmetalls verwendet.

7. Verfahren nach Anspruch 6, dadurch gekennzeichnet, daß man als Lewis Säure ein Halogenid des Bors, Aluminiums, Zinns, Zinks, Eisens oder Titans verwendet.

8. Verfahren nach Anspruch 6, dadurch gekennzeichnet, daß man als Lewis Säure ein Fluorid, Chlorid oder Bromid eines Halbmetalls oder Metalls der 3. oder 4. Hauptgruppe oder eines Übergangsmetalls verwendet.

9. Verfahren nach Anspruch 1, 2, 3 oder 4, dadurch gekennzeichnet, daß man als Lewis Säure AlCl₃, AlBr₃, FeCl₃, BBr₃, BCl₃, BF₃, SnCl₄, ZnCl₂, ZnBr₂ oder TiCl₄ verwendet.

## Claims

1. A process for the preparation of largely isomerically pure α-bisoximes of the formula Ia
R¹O-N=CR²-CR³=N-OR⁴ Ia
where the groups R¹O- and R² on the N=C bond are *cis* to one another and where the radicals have the following meanings:
R¹ is a C-organic radical;
R² is hydrogen, cyano, nitro, hydroxyl, amino, halogen or an organic radical which can be bonded to the structure directly or via an oxygen, sulfur or nitrogen atom;
R³ is hydrogen, cyano, nitro, hydroxyl, amino, halogen, alkyl, haloalkyl, alkoxy, haloalkoxy, alkylthio, alkylamino, dialkylamino or cycloalkyl,
R⁴ is hydrogen,
which comprises treating a mixture of the isomers of the α-bisoximes Ia and Ib 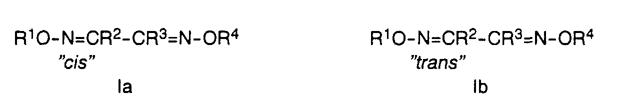 with a Lewis acid in an aliphatic or aromatic hydrocarbon.

2. A process as claimed in claim 1, wherein an α-bisoxime Ib as set forth in claim 1, wherein the groups R¹O- and R² on the N=C bond are trans to one another, is treated.

3. A process for the preparation of largely isomerically pure α-bisoximes of the formula Ia'
R¹O-N=CR²-CR³=N-OR⁴ Ia'
where the groups R¹O- and R² and the groups R³ and -OR⁴ on the N=C bonds are each cis to one another as in claim 1, wherein a mixture of the isomers of the α-bisoximes Ia' and Ib' 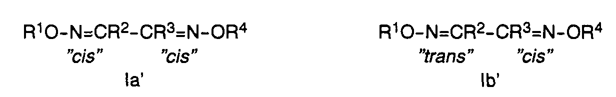 is treated with a Lewis acid in an aliphatic or aromatic hydrocarbon.

4. A process as claimed in claim 3, wherein an α-bisoxime Ib' as set forth in claim 3, where the groups R¹O- and R² on the N=C bond are trans and the groups R³ and -OR⁴ on the C=N bond are *cis* to one another, is treated.

5. A process as claimed in claim 1, 2, 3 or 4, wherein the reaction is carried out at from -40°C to 150°C.

6. A process as claimed in claim 1, 2, 3 or 4, wherein the Lewis acid used is a halide of a semimetal or metal of main group 3 or 4 or of a transition metal.

7. A process as claimed in claim 6, wherein the Lewis acid used is a halide of boron, aluminum, tin, zinc, iron or titanium.

8. A process as claimed in claim 6, wherein the Lewis acid used is a fluoride, chloride or bromide of a semimetal or metal of main group 3 or 4 or of a transition metal.

9. A process as claimed in claim 1, 2, 3 or 4, wherein the Lewis acid used is AlCl₃, AlBr₃, FeCl₃, BBr₃, BCl₃, BF₃, SnCl₄, ZnCl₂, ZnBr₂ or TiCl₄.

## Revendications

1. Procédé de préparation, à l'état d'isomères pratiquement purs, d'alpha-bisoximes de formule la
R¹O-N=CR²-CR³=N-OR⁴ Ia
pour lesquelles les groupes R¹O- et R² sont en disposition mutuelle cis par rapport à la liaison N=C, et les symboles ont les significations suivantes :
R¹ : un radical organique carboné ;
R² : l'hydrogène, un groupe cyano, nitro, hydroxy, amino, un halogène ou un radical organique, qui peut être relié au squelette directement ou par l'intermédiaire d'un atome d'oxygène, de soufre ou d'azote ;
R³ : l'hydrogène, un groupe cyano, nitro, hydroxy, amino, un halogène, un groupe alkyle, halogénoalkyle, alcoxy, halogénoalcoxy, alkylthio, alkylamino, dialkylamino ou cycloalkyle ;
R⁴ : l'hydrogène,
caractérisé par le fait que l'on traite, par un acide de Lewis dans un hydrocarbure aliphatique ou aromatique, un mélange des isomères des alpha-bisoximes la et Ib

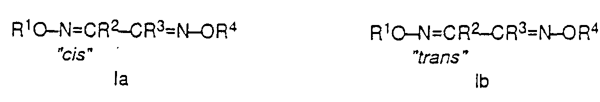

2. Procédé selon la revendication 1, caractérisé par le fait que l'on traite une alpha-bisoxime Ib selon la revendication 1 pour laquelle les groupes R¹O- et R² sont en disposition mutuelle trans par rapport à la liaison N=C.

3. Procédé de préparation, à l'état d'isomères pratiquement purs, d'alpha-bisoximes de formule Ia'
R¹O-N=CR²-CR³=N-OR⁴ Ia'
pour lesquelles les groupes R¹O- et R² et les groupes R³ et -OR⁴ sont en disposition mutuelle cis par rapport à chacune des liaisons N=C, caractérisé par le fait que l'on traite par un acide de Lewis, dans un hydrocarbure aliphatique ou aromatique, un mélange des isomères des alpha-bisoximes Ia' et Ib'

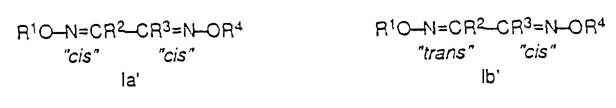

4. Procédé selon la revendication 3, caractérisé par le fait que l'on traite une alpha-bisoxime Ib' selon la revendication 3 pour laquelle les groupes R¹O- et R² sont en disposition trans par rapport à la liaison C=N.

5. Procédé selon l'une des revendications 1 à 4, caractérisé par le fait que la réaction est réalisée à des températures allant de -40 à +150°C.

6. Procédé selon l'une des revendications 1 à 4, caractérisé par le fait que l'acide de Lewis utilisé est un halogénure d'un semi-métal ou d'un métal du troisième ou du quatrième groupe principal ou d'un métal de transition.

7. Procédé selon la revendication 6, caractérisé par le fait que l'acide de Lewis utilisé est un halogénure du bore, de l'aluminium, de l'étain, du zinc, du fer ou du titane.

8. Procédé selon la revendication 6, caractérisé par le fait que l'acide de Lewis utilisé est un fluorure, un chlorure ou un bromure d'un semi-métal ou d'un métal du troisième ou du quatrième groupe principal ou d'un métal de transition.

9. Procédé selon l'une des revendications 1 à 4, caractérisé par le fait que l'acide de Lewis utilisé est AlCl₃, AlBr₃, FeCl₃, BBr₃, BCl₃, BF₃, SnCl₄, ZnCl₂, ZnBr₂ ou TiCl₄.
